# EUROPEAN PATENT APPLICATION

(11) **EP 2 042 136 A1**
(43) Date of publication of application: **01.04.2009**
(21) Application number: 08165300.8
(22) Date of filing: 26.09.2008
(51) Int. Cl.: A61F 5/445

(54) **Receptacle and method for disposing of bodily waste materials**

(30) Priority: 27.09.2007 IE 20070702
(71) Applicant: Ryder, Gerard, New Ross Wexford (IE); HOUSTON, Anne, New Ross, Co. Wexford (IE)
(72) Inventor: Ryder, Gerard, New Ross Wexford (IE); HOUSTON, Anne, New Ross, Co. Wexford (IE)
(74) Representative: Lane, Cathal Michael

(57) **Abstract**

A disposable receptacle for receiving bodily waste materials from a bodily waste discharge outlet of an active artificial excretory opening or stoma during the changing process, i.e. the removal and replacement of bodily waste material collection bags and their bodily attachment and associated wipes and cleaning materials. A receptacle having an elongate body with a top end, a base end, an intermediate portion between the top end and the base end, and formed with an internal, bodily waste material - receiving chamber; an inlet opening formed by the body, proximate to, or at, the top end thereof, the body being adapted to receive within the inlet opening bodily waste materials from a bodily waste discharge of an active artificial excretory opening or stoma during the changing process i.e. the removal and replacement of bodily waste material collection bags, its contents and their bodily attachment and associated wipes and cleaning materials to flow into or be placed into waste material -receiving chamber; and securing means for holding the bodily waste material - receiving chamber in position relative to the skin in close proximity to an artificial excretory opening or stoma. A simple yet effective device which may be easily used by a bag wearer/user which is easily disposed of by placement in waste disposal bins. It may be used to dispose of waste from an artificial excretory opening which may arise during the changing of ileostomy or colostomy bags and the safe and hygienic disposal of used ileostomy or colostomy and their contents and associated cleaning wipes and materials.

## Description

### Field of the Invention

The present invention relates to a receptacle and method of removal, replacement and disposal of bodily waste material collection bags.

Surgical construction of an artificial excretory opening or "stoma" generally takes place as an ostomy procedure. In particular the present invention concerns removal, replacement and disposal of bodily waste material collecting bags, which collect bodily waste material from an artificial excretory opening or stoma. The invention also provides for disposal of bodily attachments, discharge from artificial excretory opening or stoma and cleaning materials such as associated wipes arising from the changing process. In particular, the present invention is useful to ostomy patients, such as those who have had an ileostomy or colostomy.

### Background to the Invention

Following surgery, patients who have had a surgical construction of an artificial excretory opening such as ileostomy or colostomy patients use ileostomy/colostomy bags to collect bodily waste materials. These bodily waste materials include gases, liquids and solids. The waste material may be semi-solid faecal waste. It is desirable in any event to replace the collecting bags and bodily attachments with minimal handling from the user.

lleostomy/colostomy bags are intended for single or multiple use purposes and generally the surgical patient finds that they have to replace or empty the collection bag many times during the day. A given collection bag if designed for single use is generally not fitted with a discharge outlet. A collection bag designed for multiple use is generally fitted with a waste discharge outlet through which the waste materials collected from the artificial excretory opening can be discharged. It is not unusual to replace or empty these bags between two and ten times in any given day. A collection bag for single use will typically be replaced once to three times per day with a new bag. A collection bag designed for multiple-use may be worn for a number of consecutive days before being replaced with a new bag.

During the replacement procedure the patient or bag wearer generally has to sit on a chair or lie down on a bed to drape protective sheets such as tissues or non-woven wipes around the area of the artificial excretory opening or stoma while the collection bag (and the bodily attachments thereof such as) attachment plates, which connect the collection bag to the artificial excretory opening, are removed. The collection bag has a bodily attachment which attaches the collection bag to the patient. Often times the bodily attachment is in the form of a plate or other connector. For example such collection bags may be of the one or two-piece type as known to someone skilled in the art. The bodily attachment will have an appropriate aperture which will be in communication with the artificial excretory opening so that bodily waste materials flow into the collection bag. The bodily attachment is typically adhesive and adheres to the patients body about the artificial excretory opening.

The collection bag and its bodily attachment are normally then placed in and disposed of in a "Nappy Sack" along with any additional wipes used to clean and treat the skin in close proximity to the artificial excretory opening or stoma. This procedure is required because of the typical positioning of the artificial excretory opening on the body. Often times the opening on the body is provided at or about the stomach area and often to one side of the body. The changing procedure may be made more difficult if the artificial excretory opening or stoma is active during the changing procedure, i.e. discharges bodily waste material during the changing procedure. As mentioned above the use of protective materials such as wipes for example, tissues or non-woven wipes placed in the vicinity of collection bag and attachment only mitigate some of this difficulty. Lying down also helps with counteracting the discharge of materials through the artificial excretory opening.

Generally, therefore, conventional procedures for replacement and disposal of bodily waste material collection bags and bodily attachments are inconvenient. The problem of inconvenience for the user has been identified, and the inventors are aware of a number of proposals which seek to help a bag wearer dispose of bodily waste materials.

International Publications WO2007/000750 and WO2008/075326 (the latter published after the priority date of the present application) both to the present inventors relate to disposable receptacles for collecting waste from a collection bag for bodily waste materials.

Another arrangement is disclosed in German patent publication DE 44 18 789 Al. This invention concerns a closable container for the admission of fluids for example urine or faeces. The container is made from flexible foils welded together. A series of adhesive strips are employed, a first pair of adhesive strips of attaching to the foil container and a second series of adhesive strips for attaching to a second container. A more complex arrangement with up to six adhesive strips is also employed. Separation of certain of the adhesive strips by cutting is also disclosed. The foil bag must be put in place for collection of material from an unspecified second container. There is no mention of ostomy bags and the like and the various configurations again require manual dexterity in order to allow their use without spillage of materials.

US Patent US 5,951,532, describes a collecting bag system for human body wastes comprising a collecting bag with an inlet opening surrounded by connecting elements for connection to a body orifice. One aspect of the invention includes an extension drain to the bag which allows material making its way into the collecting bag to be continuously drained off into a large collecting bag. The invention is aimed as patients that have mobility restrictions and/or are confined to bed as evidenced by the rather cumbersome arrangement. Indeed the arrangement itself is described as a bedside arrangement for the collection of fluids. The apparatus described is unsuitable for patients that are mobile.

US Patent No. 3,825,005 describes a reusable, resealable, ileostomy or colostomy bag which may be emptied without removing the bag from the stoma of the user and is intended for use by a bedridden person. The receptacle has a complicated construction with the necessity for ribs integral with a panel to provide a seal. Again a continuous drainage arrangement is shown where a drain leads down to an accumulator which collects the continuously draining material from the bag. Again this arrangement is not suited for use by mobile collection bag wearers.

US Patent 3,841,332 relates to an enterostomy drainage bag having front and rear walls of flexible moisture-proof material forming a fluid collection chamber therebetween. The bag is intended for continuous drainage use for a person who remains in one place being primarily aimed at patients confined to bed or for night time use. A flexible tube is required to drain the bag which can be connected to a larger remote collection container. This bulky equipment is not considered portable.

UK patent application No. GB 2,258,399 and US patent no, 4,285,076 each describe a fixed plumbed device for emptying and cleaning a collection bag for excreta, while US patent no, 5,671,485 discloses a device that can be fitted onto a toilet seat to support a colostomy bag. Other ostomy pouches, which may be disposed through toilets, are described in Canadian patent application No. 1,320,324 and US patent No. 5,769,831. US patent No. 4,930,942 describes a wrapping or enclosure into which flexible soft objects such as ostomy pouches can be placed. The material of the wrapping or enclosure becomes slimy on contact with water in the toilet bowl thus allowing for flushing. An inner and outer bag arrangement is described in the German patent application DE 19,519,069,

US Patent No 5,938,647 to Welland Medical Limited discloses a biodegradable flushable ostomy bag liner. The liner is designed to be used in a two-bag ostomy bag system. The liner is adapted to fit into the outer bag and collect the waste material. When full the liner can be removed from the outer bag and disposed of by flushing in a toilet. The outer bag may have to be disposed of separately for example in a dustbin.

US 4,387,713 describes a disposable discharge collector to receive the drain opening of a stoma pouch and having a wiper. EP 1 169 984 describes an ostomy bag with a sealant device on the drain thereof. GB 2 414 677 describes a drainable pouch for an ostomy bag with an opening and closing mechanism. US 6,116780 describes a disposable toilet receptacle.

One commercial toilet flushable pouch (similar to the device described in U.S. Patent No. 5,938,647) is available from Welland Medical Limited in the UK. Their product sold under the trade mark Flair Xtra™ is an inner liner pouch which is designed to fit within the bag worn by the user. The pouch is designed and arranged to collect the bodily waste materials and provide a barrier between those materials and the bag. None of the bodily waste materials therefore comes into direct contact with the inner of the bag. Additionally, the bodily waste materials can be removed by removing the inner pouch without contamination of the bag.

This means however that the inner pouch must be of a relatively complex design as it must be adapted to fit to the artificial opening of the body in the same way as the collection bag. This is necessary in order to avoid contamination of the collection bag with bodily waste material.

Notwithstanding the foregoing it is desirable to provide alternatives for users of collection-bags.

### Summary of the Invention

It is an object of the present invention to provide an improved receptacle and method for (replacement and) disposing of bodily waste material collection bag and bodily attachment that overcomes some of the problems of the prior art above such as difficulties associated with an active artificial excretory opening or stoma. It is a further object of the present invention to provide a receptacle that is both portable and simple and convenient for the end user to employ.

The present invention provides a receptacle and method for disposing of bodily waste material collection bags and their associated bodily attachments.

The present invention provides a disposable receptacle for receiving bodily waste material collection bags and a bodily attachment for the collection bag in the process of removal and replacement of bodily waste material collection bags by new bags and attachments to/from an artificial excretory opening, the receptacle comprising:
an elongate body with a top end, a base end, an intermediate portion between the top end and the base end, and formed with an internal, bodily waste material-receiving chamber;
an inlet opening formed on the body, proximate to, or at, the top end thereof, the body being adapted to receive within the inlet opening:
   (i) bodily waste discharge from an active artificial excretory opening;
   (ii) a used bodily waste material collection bag;
   (iii) used bodily attachments which connected the used bodily waste material collection bag to the artificial excretory opening; and a securing means for holding the bodily waste material-receiving chamber in position relative to an artificial excretory opening for the removal and replacement of bodily waste material collection bags, its bodily attachments and their disposal into the bodily waste material-receiving chamber.

In particular the present invention provides a disposable receptacle for receiving bodily waste materials from a bodily waste discharge from an artificial excretory omening orstoma during the changing of a bodily waste material collection bag and the bodily attachment, to collect and retain bodily waste materials from a user's body, the receptacle comprising:
(i) an elongate body with a top end, a base end, an intermediate portion between the top end and the base end, and formed with an internal, bodily waste material-receiving chamber;
(ii) an inlet opening formed on the body, proximate to, or at, the top end thereof, the body being adapted to receive within (and through) the inlet opening the bodily waste material collection bag and the bodily attachment into the bodily waste material receiving chamber and (iii) loop handles formed from extension of the body of the receptacle, proximate to the top of the receptacle and bounding the inlet opening formed in (ii) above; (iv) securing means for holding the bodily waste material-receiving chamber in position and discharge of the bodily waste materials into the bodily waste material-receiving chamber from the artificial excretory opening or stoma; (v) a securing means for holding the receptacle relative to the end-users body and in close proximity to the bodily attachment of the bodily waste collection bag and the artificial excretory opening or stoma.

The receptacle of the invention may as set out in the alternative definitions herein (including in the claims) or any combination thereof.

The present invention therefore provides a simple yet effective device which may be easily used by a bag wearer/user. It is easily disposed of also. The disposable receptacle of the invention will thus remain in place when the collection bag and any bodily attachment for the collection bag is removed and will collect any bodily waste materials from the excretory opening discharged after the collection bag is removed and before a new collection bag is fitted. The collection bag (any contents thereof) and any bodily attachment are all placed in the disposable receptacle. The disposable receptacle can then be disposed of. Any cleaning materials such as wipes can be placed in the disposable receptacle also.

A user of the disposable receptacle does not have to lie down then to change the collection bag as the receptacle of the invention will collect any bodily waste materials which are discharged even after the collection bag is removed.

In a one-part bag the bodily attachment is (permanently) on the collection bag. In a two-part bag the bodily attachment and the bag are releasably connectable to each other.

The present invention provides a disposable receptacle for receiving bodily waste materials, bodily waste material collection bags, its bodily attachment and associated wipes involved in the process of removal and replacement of bodily waste material collection bags (colostomy and ileostomy bags) by new bags and attachments to/from an artificial excretory opening or "stoma", the receptacle comprising:
an elongate body with a top end, a base end, an intermediate portion between the top end and the base end, and formed with an internal, bodily waste material-receiving chamber;
an inlet opening formed on the body, proximate to, or at, the top end thereof, the body being adapted to receive within the inlet opening the bodily waste discharge from an active artificial excretory opening or "stoma"; and
an inlet opening formed on the body, proximate to, or at, the top end thereof, the body being adapted to receive within the inlet opening a used bodily waste material collection bag; and
an inlet opening formed on the body, proximate to, or at, the top end thereof, the body being adapted to receive within the inlet opening used bodily attachments which connected a used bodily waste material collection bag to artificial excretory opening or "stoma"; and
a securing means for holding the bodily waste material-receiving chamber in position relative to an artificial excretory opening or "stoma" for the removal and replacement of bodily waste material collection bags and its bodily attachments and their disposal into the bodily waste material-receiving chamber.

The securing means may be for securing the waste material receiving chamber: (i) relative to the artificial excretory opening or stoma; (ii) relative to the skin in close proximity to the artificial excretory opening or stoma; (iii) relative to the bodily attachment of a bodily waste material collection bag, where such collection bags are of the one or two piece type as known to someone skilled in the art.

The securing means described above may enable the bodily waste material receiving chamber to be secured (and supported) in a usable position. This allows the end user to have both hands free to arrange the removal and replacement and disposal of the collection bag and its bodily attachment and any associated wipes and any waste material.

Any form of such securing means may be provided, including press-fit arrangements and those such as certain self-securing arrangements. The securing means for securing the waste material receiving chamber relative to the artificial excretory opening or stoma may be referred to as a "collection securing means".

In one arrangement of the present invention it is desirable that the receptacle body is self-securing, for example to the skin in the vicinity of the bodily waste collection bag bodily attachment and the artificial excretory opening or stoma. In this respect the receptacle body may be provided with adhesive for self-adhering to the skin in the vicinity of the artificial excretory opening. For example a peel-off tab may be provided over an adhesive patch on the receptacle. Removal of the peel-off tab exposes the adhesive. The adhesive can then be utilised to fulfil the securing function. Additionally or alternatively the securing means may take the form of a belt attachment to the body of the receptacle, which would tie around the body of the end-user.

In all of these arrangements the potential spillage of materials from an active artificial excretory opening or stoma may be avoided.

Such self-securing of the receptacle body helps to ensure that no bodily waste materials are accidentally spilled during the removal and replacement process of the bodily waste material collection bag and its bodily attachment. Once the bodily waste material collection bag, its bodily attachment and any wipes used in the process have been taken into the receptacle, the receptacle may be disposed of.

The present inventors envisage a receptacle which may be used conveniently by a bag wearer. In the embodiments of the present invention the receptacle forms a container which is designed to hold the bodily waste material collection bag its bodily attachment and any disposable wipes etc, received during the removal procedure. The bodily waste material collection bag, its bodily attachment and associated wipes are collected for subsequent disposal with the receptacle.

In all embodiments of the present invention it is desirable that the receptacle is disposable through the normal channels for disposing of domestic waste material. In this respect it is desirable that the receptacle is constructed of a material which will degrade naturally over time. Such materials as may be disposed of by means of normal channels for disposing of domestic waste materials, are well known to the persons skilled in the art. Typical materials which may be used include degradable fibrous materials such as those based on cellulosic materials for example paper. Other biodegradable materials may also be employed such as biodegradable plastics. Suitable materials include (biodegradable) starch-based materials. Materials such as polyethylene or polypropylene may also be used. For example a "nappy sack" (diaper sack) type material may be employed or a combination of non-woven material and plastic or copolymer material for example where the polymers are ethylene and propylene may be used. One suitable material is LDPE (low density polyethylene).

The disposable receptacle of the invention may be used as an emergency toilet for example were a collection bag is unavailable to collect the material (e.g. is already full) or where toilet facilities are unavailable.

The disposable receptacle can be provided with suitable absorbent materials for example super absorbent material (often referred to as "SAM"). This material is particularly useful when the receptacle is to be used as a toilet. Such SAM material can comprise many materials including polyacrylate absorbent materials and be in any desired form such as in the form of absorbents granules. The SAM may be selected so that it (together with absorbed fluid faecal material) is transformed into a gel.

In one arrangement the SAM can be in the form of granules held in the receptacle for example in a pack. For example it may be held in a suitable outer layer such as a soluble outer layer. The SAM can be provided in the form of a sheet (typically with multiple layers) for example a sheet comprising one or more polyester layers and a polyolefin adhesive. In such a form the SAM has the appearance of a tissue.

Where the receptacle of the invention is secured to a user's body using adhesive then it is desirable to use a hypoallergenic adhesive.

The bag is designed as a changing bag for colostomy bags. Many colostomy bags are attached to a patient about an artificial excretory opening using a bodily attachment. Such bodily attachments are often times secured by an adhesive.

To change a collection bag a user removes the collection bag which typically involves detaching the adhesive patch of the bodily attachment from the body. The user then cleans away any remaining adhesive and inspect the skin for damage and medicate as required. The faecal discharge from the stoma of users in particular ileostomy patients is very fluid and the stoma may discharge faecal matter while the area around the stoma is being cleaned or inspected. This can lead to soiling of the patients, patient's clothing, bedding or floors. The receptacle of the present invention is designed to form a seal around the area of the colostomy bag attachment point and catches any discharge from the stoma. It is also sized to accept the used colostomy bag, the colostomy skin attachment and any cleaning materials used such as wipes. It allows the user to clean the attachment point without fear of splashing or soiling himself/herself.

It will be appreciated that the receptacle must be sufficiently resistant to degradation for a sufficient period of time in contact with the bodily waste materials to prevent premature disintegration thereof and potential resultant contamination from leaking bodily waste materials. Suitable materials for the receptacle include plastics films, for example plastics films with a paper backing, such as the materials used in motion sickness bags.

Generally, it is desirable that the receptacle is collapsible from an expanded state for use to receive the discharged waste materials, the collection bag and its bodily attachment and associated wipes to a collapsed state for storage. In this respect the receptacle will be constructed of a material which allows it to be moved between the collapsed and expanded states. The materials mentioned above are suitable in this respect also.

It is desirable that the collapsed state of the receptacle is a substantially flat condition thereof. This means that the receptacle can be discreetly and easily carried by a user of a collection bag. The receptacle is thus very easy to use at various locations. Users do not need to concern themselves with having specialised equipment available for removal and replacement of the bodily waste material collection bag, its bodily attachments and any associated wipes.

It is further desirable that the receptacle is provided with one or more constrictions between the top end and the base end thereof. The constriction(s) prevent (full) expansion of the receptacle at a position intermediate the top end and the base end. More particularly the restriction(s) will counteract any tendency of the receptacle to sag when a collection bag or other contents are held therein. Sagging can cause a tendency of a mouth of the receptacle to close.

The constriction(s) may be formed by a weld e.g. one or more spot welds, for example spot welds on left and right sides of the receptacle.

The constriction(s) may be formed by joining a front wall of the receptacle to a rear wall thereof. The constriction(s) will not prevent the collection bag or any other item disposed of moving down the material-receiving chamber. Instead the constrictions will join parts of the receptacle so the part of the receptacle attached to the user will give support to an opposing side (which side is not generally attached to the user).

It is desirable that the elongate body of the receptacle does become narrower in a direction from the top end thereof towards the base end thereof, so that it can receive the bodily waste collection bag its attachments and any associated wipes more easily.

It will be appreciated that the receptacle may be provided with a closure for its inlet opening. Such a closure again mitigates against spillages etc. Such a closure may be provided by a folding piece (for example a flap) on the receptacle body. The closure may be provided with securing means (of the self-securing type in particular) to hold it closed. The closure may fold across the inlet opening to provide a closure therefore. It is also envisaged that loop handles may be used to tie up the opening of the bag to prevent spillage.

In one arrangement, the receptacle has opposing walls which are joined by interposed collapsible wall portions so as to form the elongate body thereof. The collapsibility of the collapsible wall portions may be provided by at least one longitudinal fold line running along each of the collapsible wall portions. Wall portions which collapse by folding about the fold line may be provided. Suitably, a plurality of fold lines are provided on each of the collapsible wall portions. Where a plurality of fold lines are provided it is desired that folding occurs in what is known as a fan-type, a fluted, a bellows-type or accordion-type folding arrangement. In such an arrangement the parts of the wall portions between successive fold lines collapse or stack upon each other when a compression force to the collapsed state is experienced. Desirably an odd number of fold lines are provided for example 3, 5, 7 or 9..

In, an alternative embodiment, the side walls may be configured in such a way so that they do not have fold lines. For example side walls may be formed by sealing the edges of the opposing walls (front and rear walls). Suitably the side walls may be formed by heat sealing the edges of the opposing walls.

Indeed it is desirable that a resting state of the receptacle is its collapsed state. In such a case, a user must exert a force to move the receptacle to its working configuration.

It is desirable that the securing means is self-securing to the skin in close proximity to the artificial excretory opening. In this respect it may be provided with adhesive for self-adhering to be attached to the skin. For example a peel-off tab or cover may be provided over an adhesive patch which is attached to the skin in close proximity to the artificial excretory opening or stoma.

The adhesive patch may be shaped to describe "-" a horizontal seal below the bodily attachment of the bodily waste collection bag, or "U" or a semi-circle around the bodily waste disposal collection bag bodily attachment, and is also attached to the skin in close proximity to the bodily waste collection bag bodily attachment and the artificial excretory opening or stoma prior to the commencement of the removal and replacement procedure. Removal of the peel-off tab exposes the adhesive. The securing means may include "|" shapes (vertical seals each side of a bodily attachment for a collection bag). The securing means may be provided in the form of an adhesive strip.

Generally the receptacle securing means will be an adhesive strip constructed from a layer of adhesive with a peel-away cover which may be attached to and slightly below the rear opening of the receptacle (running in the horizontal plane of the bag in the case of the "-" securing means). In the case of the "U" the securing means may take the form of die cut strip or strips which attach to the rear of the loop handles to form a vertical seal and attach below the rear opening of the receptacle to form a horizontal seal.

Generally, it will be that side of the receptacle securing means which faces away from the receptacle inlet that is secured to the skin.

In some embodiments of the current invention it may be desirable to provide a belt which would tie around the body of the end user to assist in supporting the weight of the bodily waste collection bag, its bodily attachment and any cleaning materials. In such embodiments the adhesive securing means would be arranged to form horizontal and vertical seals in close proximity to the bodily attachment of the bodily waste collection bag. However the adhesive securing means may not be sufficient on its own to support the weight of the bodily waste collection bag, its bodily attachment and cleaning materials used in the changing procedure. The belt helps in such circumstances.

Desirably, when the receptacle is secured to the skin in close proximity to the artificial excretory opening or stoma by a securing means the end user may sit on a chair or edge of a bed and remove and replace the collection bag and its bodily attachment, without the risk of spillage.

It will be appreciated that the receptacle is of a length to allow it to receive the bodily waste material collection bag, its bodily attachment; associated wipes and bodily waste material which discharges from an active artificial excretory opening or stoma or any combination of the above.

The present invention also relates to a method for disposing of bodily waste materials comprising the steps of:
attaching a receptacle to the skin in close proximity to a bodily waste material collection bag, its bodily attachment and an artificial excretory opening, so that an inlet opening of the receptacle receives the bodily waste discharge from an active artificial excretory opening;
placing the bodily waste material collection bag and its bodily attachments into the receptacle while the receiving receptacle is attached to the users body;
placing wipes associated with the changing of the bodily waste material collection bag and its bodily attachment into the receptacle while the receiving receptacle is attached to the users body; and
placing any combination of the contents thereof in a receptacle while the
receiving receptacle is attached to the users body.

The present invention also provides a kit comprising:
a receptacle with an elongate body with a top end, a base end, an intermediate portion between the top end and the base end, and formed with an internal bodily waste material-receiving chamber; an inlet opening formed on the body, proximate to, or at, the top end thereof, the body being adapted to receive within the inlet opening the bodily waste discharge from an active artificial excretory outlet; a bodily waste material collection bag, its bodily attachment and the contents of the bodily waste collection bag; and associated wipes, as used in the changing of bodily waste material collection bags, for placing into the receptacle while the receptacle is attached to the skin in close proximity
to an artificial excretory opening.

The kit may additionally comprise one or more of a replacement collection bag, wipes, or cleaning materials. The kit may comprise a plurality of one or more of same. The kit may additionally include a plurality of replacement bags.

The invention extends to a receptacle substantially as described herein with reference to the drawings.

### Brief Description of the Drawings

The present invention will be more clearly understood with reference to the accompanying drawings in which:

**Figure 1** is a rear plan view of one embodiment of a receptacle of the present invention having securing means and loop handles and optional belt as securing means, in a closed or collapsed configuration;

**Figure 2** is a perspective view of the receptacle of Figure 1 in an open position or expanded configuration;

**Figure 3** is a perspective view of the receptacle of Figure 1 and its securing means positioned relative to the bodily waste collection bag, its bodily attachment and the artificial excretory opening or stoma;

**Figure 4** is a perspective view of an arrangement very similar to that of Figure 3; and

**Figure 5** shows a rear plan view of an alternative embodiment of a receptacle of the invention similar to that of Figure 1.

**Figure 6** shows a perspective view of the receptacle of Figure 5 in an expanded configuration.

### Detailed Description of the Drawings

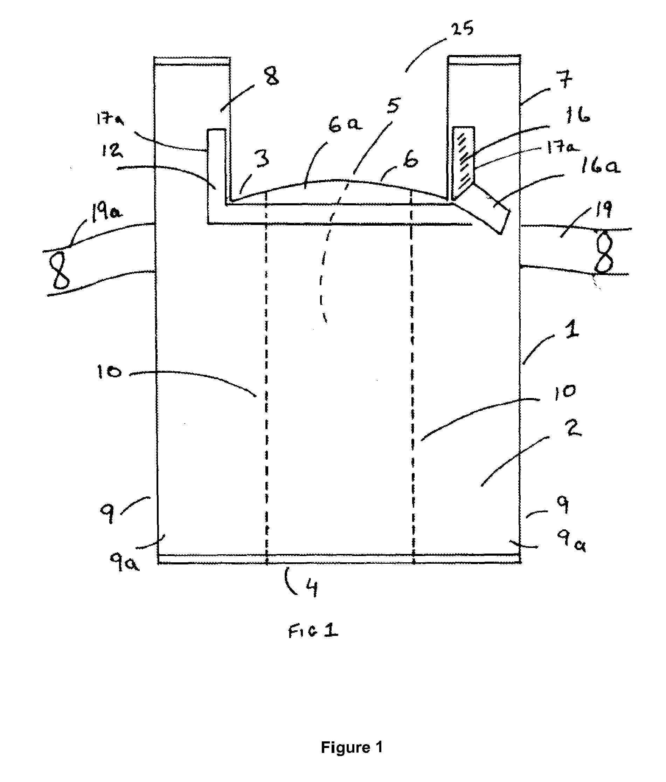

Figures 1 to 3 show a disposable receptacle of the present invention in the form of a bag 1,and having a securing means 12. The bag 1 has an elongate body 2. The elongate body 2 has a top end 3 and a base end 4. The bag 1 is formed with an internal waste material-receiving chamber 5 formed between the top end 3 and the base end 4. The bag 1 is formed with loop handles 7,8.

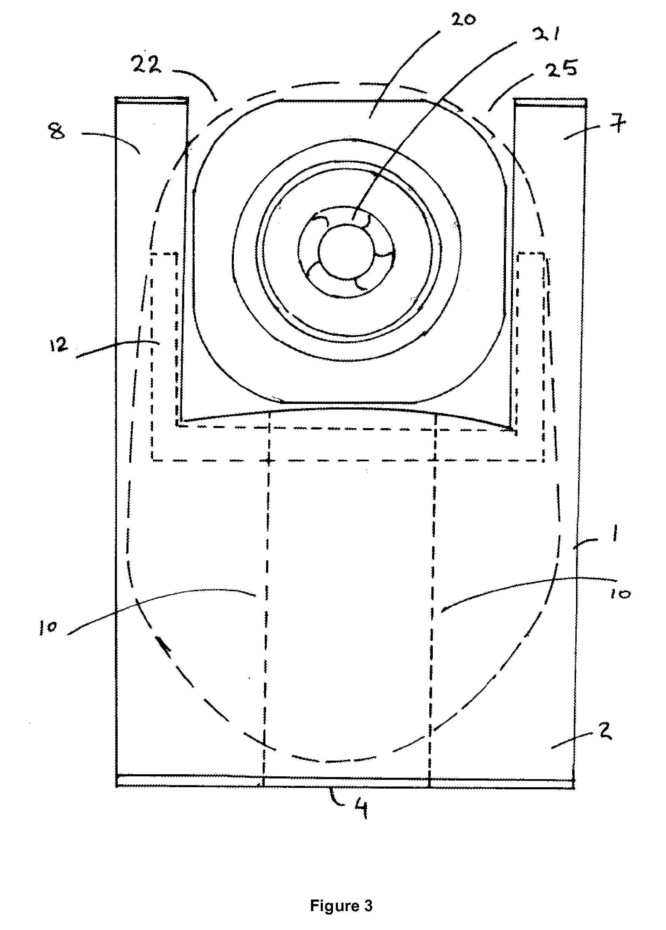

The elongate body 2 and waste material-receiving chamber 5 are of suitable dimensions so that they can receive waste material associated with the removal and replacement of a bodily waste material collection bag 22 (such as a colostomy or an ostomy collection bag) and its contents (see **Figure 3**). For example, the internal dimensions of the waste material-receiving chamber 5 are adequate to receive the volume of a bodily waste material collection bag, its bodily attachment 20, any associated wipes and any discharge from an active artificial excretory opening or stoma 21.

The bag part 1 of the disposable receptacle of the present invention can be manufactured in a number of sizes. For example smaller sized disposable bags 1 may be suitable for use by children or persons with a requirement for small discharge collection bags. Whereas an adult user may require a larger bag part 1 to a disposable receptacle as the volume of waste that their discharge collection bag holds will be greater. Typical maximum dimensions of the disposable receptacle are in the region of 520 mm x 295 mm x 180 mm with loop handles of dimensions 160 mm x 60 mm and a cut out of 160 mm x 152 mm. Other dimensions such as 350 mm x 295 mm x 180 mm with loop handles of dimensions 130 mm x60 mm and a cut out of 130 mm x 152 mm. Minimum usable dimensions are in the range of 310 mm x 180 mm x 100 mm with loop handles of dimension 100 mm x 35 mm and a cut out of 100 mm x 110 mm. Dimensions of the receptacle will typically range between these maximum and minimum dimensions. Disposable receptacles of the present invention may be marked, for example colour coded to indicate the maximum volume the waste material-receiving chamber 5 can accommodate.

The bag part 1 of the disposable receptacle has an inlet opening 6. The inlet opening 6 is formed by a mouth 6a about the top end 3 of the elongate body 2. In the embodiments the inlet opening 6 is at the top end 3 of the elongate body 2 but of course could alternatively be located proximate thereto. The inlet opening 6 is adapted to receive waste material as stated above. Preferably, the inlet opening 6 is configured so that when the receiving chamber 5 has received the bodily waste material collection bag, its bodily attachment, any associated wipes and any discharge from an active artificial excretory opening or stoma, the receptacle can be folded over to make a leakproof seal. It may be desirable to have applied a dedicated peel away cover and adhesive strip to achieve this seal. Notwithstanding this, the adhesive securing means may be used to achieve this seal. It may be desirable to tie up the loop handles to seal the opening. In such a configuration, the risk of spillage of the waste material is minimized.

The securing means 12 illustrated in Figure 1 is a self-securing means. In the embodiment, the securing means 12 takes the form of a strip 16 of adhesive (covered by a peel-off cover 16a) attached to the rear of the loop handles and attached slightly below the rear of opening 6 of the receptacle proximate to the top end 3 of the receptacle. The adhesive patch/ patches 16 may be used to secure the top end 3 of the receptacle to the skin in close proximity to the collecting bag bodily attachment and artificial excretory opening or stoma. In particular, in the embodiment illustrated, the securing means 12 is elongate so that the adhesive strip 16 (will extend about) and form a "U" shape about the collection bag bodily attachment (see Figure 3). The parts (vertical portions 17a) of adhesive strip 16 attached to (extending along) loop handles 7 & 8 form a vertical seal of the "U", while the part (horizontal portion 17b) of adhesive strip 16 attached below the rear of opening 6a forms the bottom seal of the "U". This shape helps to provide extra grip (security) and prevent leakage of faecal matter. The securing means 12 is desirably a (tacky) low strength adhesive. The adhesive strip 16 may be sized such that it is strong enough to secure the receptacle to the users body and support the weight of a used collection bag, its bodily attachment and discharge from an active artificial excretory opening or stoma whilst allowing the end user to easily remove the adhesive strip 16 from the skin. Suitable adhesives for use with any embodiment of the invention include: double sided hypoallergenic adhesive RX268S manufactured by Scapa Medical, Med 3044 manufactured by Avery Dennison, ArCare 7396 manufactured by Adhesives Research Limited.

In the embodiment shown in Figure 1, the securing means 12 is shown as a die-cut one-piece adhesive strip. However the attachment (securing means) may be made up of separate pieces for example 2, or 3 pieces.

The length of the receptacle will vary depending on the size of the end user. For example a child would require a smaller length (smaller capacity) compared to an adult. Typically, the length of the receptacle would be in the region of 520mm to 310mm. The product may be colour coded to indicate its length. Once a new bodily waste material collection bag 22 and its bodily attachment 20 have been attached to the skin in close proximity of the artificial excretory opening or stoma, the receptacle or bag 1 and its content can be removed for disposal. The receptacle or bag 1 may be disposed of by any method such as being placed in a bin.

The desired way of disposing of a used receptacle is by placing it in a bin. The receptacle is preferably made of any suitable biodegradable material which can be disposed of by landfill or composting in an environmentally friendly way. Desirably the receptacle is made of a cellulosic type material. The material of which the receptacle is constructed must have sufficient resilience so that it does not disintegrate when the waste material enters the receptacle. Suitable materials for manufacturing the receptacle include treated cellulosic material, polyethylene or polypropylene films, plastics films with a paper backing, or a copolymer for example where one of the polymers is ethylene and the other is propylene.

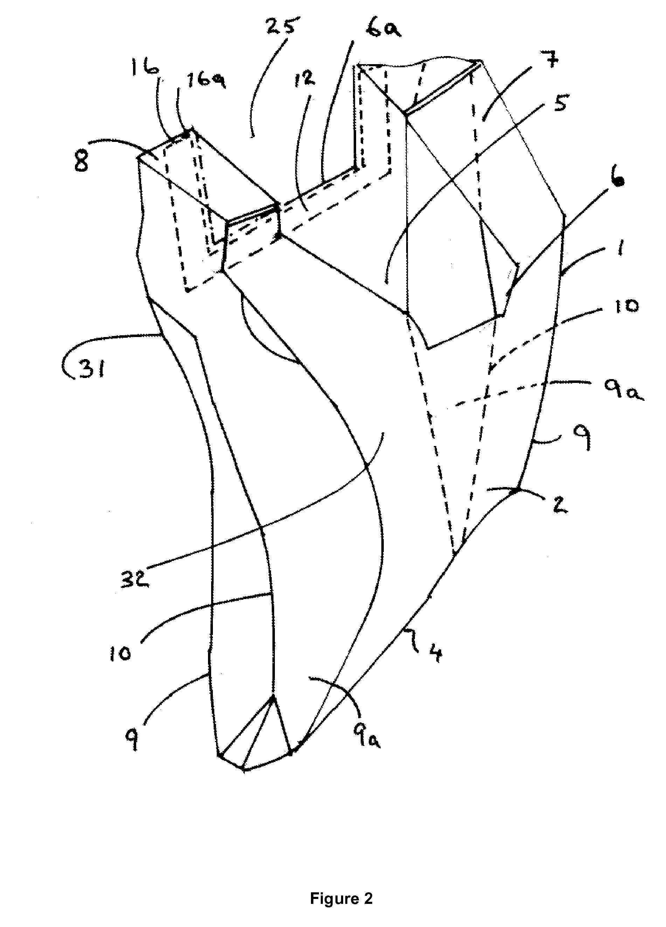

The receptacle may be an immediately disposable device as described above or may be utilised a small number of times. The receptacle may also be a portable device. The receptacle preferably has two states: a collapsible state and an expanded state. The collapsible state may be used for storage. The expanded state shown in **Figure 1** (rear view) and **Figure 2** (perspective view from front, side and above) is used for receiving the waste material from the collection bag 22 its bodily attachment 20 and waste discharged from an active artificial excretory opening or stoma 21 during the changing process. Desirably, the receptacle is constructed from suitable material that allows it to be moved between the collapsed and extended states. Suitable materials includes those listed above. Desirably the receptacle has opposing walls 9 such as front 32 and rear 31 walls. The opposing walls 9 may be joined by interposed collapsible wall portions 9a. The collapsibility of the wall portions 9a may be provided by longitudinal fold lines 10 running along each of the collapsible wall portions 9a. In the embodiment shown in **Figures 1** **and** **2**, the collapsible wall portions 9a have one longitudinal fold line 10. The number of fold lines 10 in the collapsible wall portions 9a will generally be an odd number.

In this embodiment the receptacle or bag 1 is fitted with a belt in the form of belt portions 19 & 19a which may be used as an additional securing means to support the receptacle and maintain its position relative to the bodily waste material collection bag, its bodily attachment and the artificial excretory opening or stoma. The belt is secured about the body of the user for additional support for the bag 1.

**Figure 2** illustrates the receptacle 1 in the open position, suitable for receiving a bodily waste collection bag 22, its bodily waste attachment 20, bodily waste material from an active artificial excretory opening or stoma 21 and any wipes and cleaning material that arises from the removal and replacement of a bodily waste material collection bag 22 and its bodily attachment 20. The bag is shown in the open position.

In this embodiment the securing means 12, adhesive strip 16 and peel away cover 16a are shown as dashed lines to the rear of the receptacle. The securing means 12 is shown describing a "U" shape about a cut-out 25. In the embodiment the cut-out 25 is formed between the top of the bag and the loop handles 7,8 The end-user opens the mouth 6a of the receptacle inlet 6 and causes the front and rear walls 32 and 31 to separate showing the receiving chamber 5 of receptacle 1. In this process loop handles 7 & 8 will tend to flatten until the they are restrained by the adhesive strip 16 attached to loop handles 7 & 8. This stretches the opposing walls 9 & 9a and the fold line 10 to allow the receptacle receiving chamber 5 to accommodate the bodily waste collection bag 22, its bodily waste attachment 20, and any bodily waste material from an active artificial excretory opening or stoma 21.

**Figure 3** illustrates the receptacle or bag 1 in the closed position, and positioned relative to the receiving bodily waste collection bag (dashed outline 22), its bodily waste attachment 20 (which takes the form of a plate with an aperture defined therein,) and artificial excretory opening or stoma 21. (The user has not been shown for clarity of illustration). In this embodiment the securing means 12 on loop handles 7 & 8 describe a "U" shape around cut out 25 and bodily attachment 20 and the artificial excretory opening or stoma 21. The outline of the bodily waste material collection bag 22 is shown in dashed outline for clarity.

Before replacing a collection bag 22 a user places the bag 1 in a position such as that shown in Figure 3 where the bag 1 is attached to the user about a stoma. Typically a user will be in a sitting or standing position so that the collection bag 22 and the bag 1 of the invention have the substantially vertical orientation shown in Figure 3. The bag 1 may be attached to a user utilising the securing means 12 by removing off peel-off cover strip 16a to expose adhesive 16 which can then be used to adhere the bag 1 in place. The cut-out 25 allows the bag 1 to extend about the stoma 21 on three sides. If present and/or as desired the belt 19,19a may additionally be utilised to hold the bag 1 in place by securing the belt about the user. The collection bag 22 (while still attached to the user) is placed into the bag 1 to the extent possible while the bodily attachment 20 is still attached to the user. This means that the bag 1 is positioned to receive the collection bag 22 into its material receiving chamber 5. If the collection bag 22 is a two-part bag (a bag part separable from the attachment 20) the bag may be released into the bag 1 and then the bodily attachment 21 removed and placed into bag 1. Alternatively the user can choose to remove the collection bag and its bodily attachment together or if the attachment is to be used for a new bag it may be left in place. If the collection bag 22 is a one-part bag then the user will remove the collection bag and its bodily attachment together. When the collection bag is removed the stoma may still be active and the bag 1 will catch any material discharged by the stoma. The area can then be cleaned and/or medicated as necessary. Any materials used in this process, for example wipes, may additionally be placed into the chamber 5 of the bag 1. A new bodily attachment 20 may be fitted if required. A new collection bag 1 may then be attached to the newly fitted bodily attachment or directly to the user. The disposable receptacle 1 can be closed if desired. It is removed from the user and disposed of as appropriate.

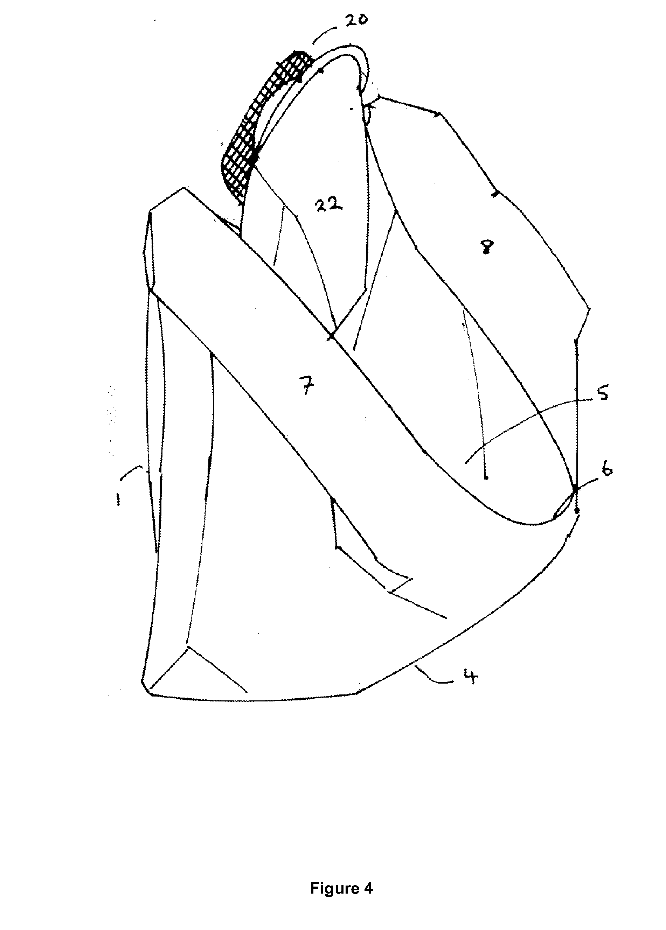

**Figure 4** shows a perspective view of an arrangement very similar to that in Figure 3 with the collection bag 22 still in a position where it is attached to a user. In the embodiment the bodily attachment 20 includes an (annular) adhesive flange which attaches to the users body about the stoma 21. As can be seen the bag 1 of the invention is positioned to receive the collection bag 22 and any discharge from the stoma when the collection bag is removed. The inlet opening 5, in the embodiment of the bag 1 in Figure 4 extends a substantial way down the bag 1 to a position closer to the base end 4 of the bag 1.

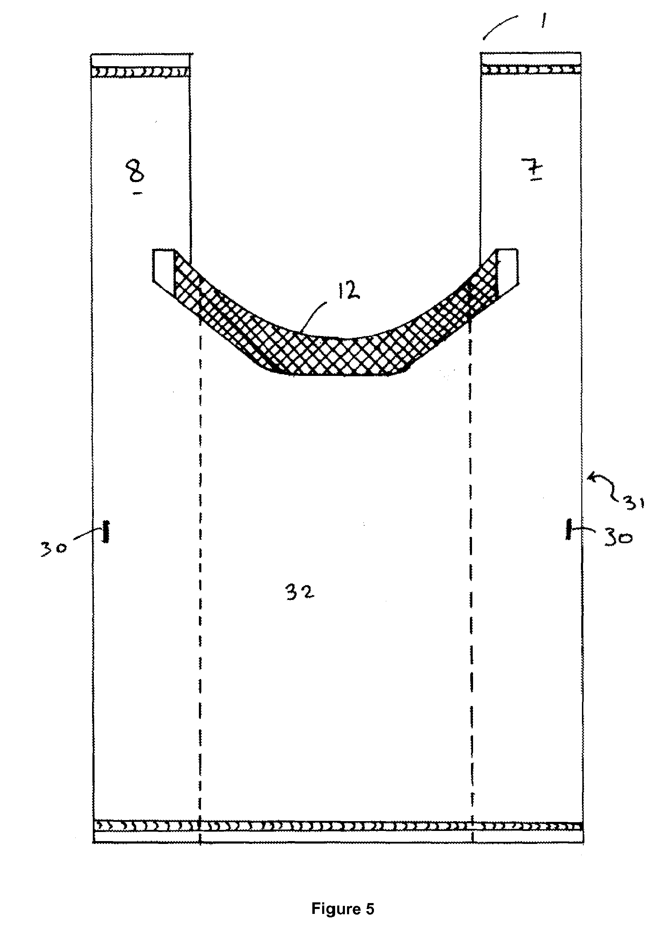

**Figure 5** shows an additional embodiment of a receptacle 1 of the invention. In the embodiment the securing means 12 has a generally semi-circular shape.

Also the embodiment includes attachment points in the form of welds 30 which may be in the form of spot welds. These form constriction points restricting the extent that the receptacle can open at a position between the top end and base thereof. Welding may be achieved by heat-welding. The attachment points 30 attach the front wall 32 to the rear wall 31 of the bag. This means that when the rear wall is attached to a user the front wall is supported by the rear wall. This mitigates against sagging of the receptacle. It is to be noted that the attachment points are in addition to any connection between the front and rear walls, for example via a side wall or where a front wall and rear wall directly join at their respective sides to form the receptacle (for example where there are no side walls).

The sidewalls of the receptacle 1 of the invention are free to expand down as far as the attachment points 30, but are constrained at the attachment points 30. The sidewalls are free to expand below the attachment points 30 (the bottom of the bag). The purpose of the attachment points is to stop the bag distorting when a full colostomy bag is placed in the disposable receptacle. The attachment points do not however prevent contents from reaching the base of the receptacle.

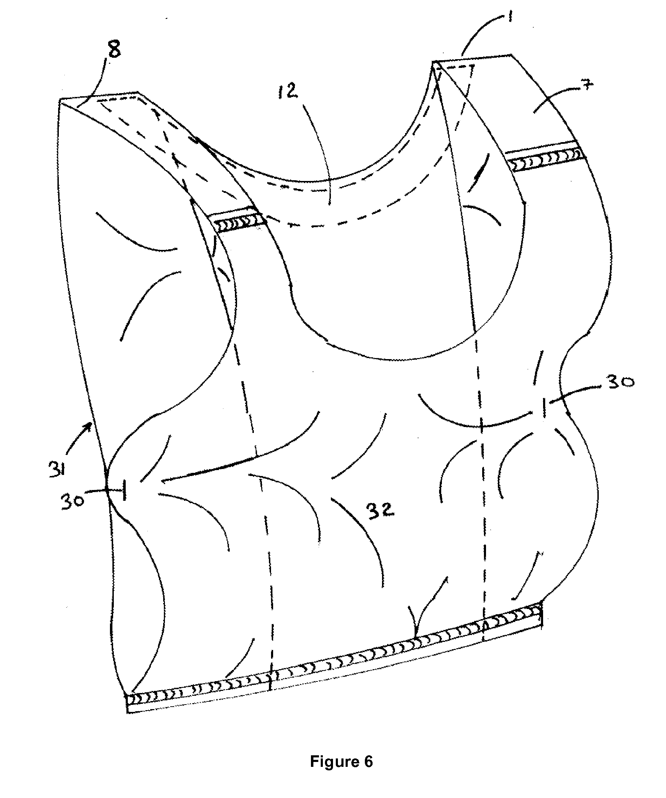

**Figure 6** shows a perspective view of the receptacle 1 Figure 5. In the configuration of Figure 6 the receptacle 1 is in an expanded configuration. In the expanded configuration it is ready for receiving, or may already hold, a collection bag and any bodily attachment therefor and any wipes associated with the cleaning process. As can be seen from Figure 6, the attachment points 30 form a restriction against full expansion of the bag. In particular, It can be seen that the front wall 32 is attached to the rear wall 31 so as to from a throat in the material receiving chamber. This helps to keep any contents in the receptacle 1 close to the rear wall 31. This stops the receptacle 1 from sagging when contents are in the base thereof. Direct attachment is one suitable way of forming the attachment points, and welding is one convenient method. It will be appreciated that other attachment points can be created for example using a bridging element that spans across the receptacle for example from the front wall to the rear wall.

The words "comprises/comprising" and the words "having/including" when used herein with reference to the present invention are used to specify the presence of stated features, integers, steps or components but do not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

## Claims

1. A disposable receptacle for receiving bodily waste material collection bags and a bodily attachment for the collection bag in the process of removal and replacement of bodily waste material collection bags by new bags and attachments to/from an artificial excretory opening, the receptacle comprising:
an elongate body with a top end, a base end, an intermediate portion between the top end and the base end, and formed with an internal, bodily waste material-receiving chamber;
an inlet opening formed on the body, proximate to, or at, the top end thereof, the body being adapted to receive within the inlet opening:
(i) bodily waste discharge from an active artificial excretory opening;
(ii) a used bodily waste material collection bag;
(iii) used bodily attachments which connected the used bodily waste material collection bag to the artificial excretory opening; and
a securing means for holding the bodily waste material-receiving chamber in position relative to an artificial excretory opening for the removal and replacement of bodily waste material collection bags, its bodily attachments and their disposal into the bodily waste material-receiving chamber.

2. A receptacle according to Claim 1 wherein the securing means is an adhesive strip with a peel away cover over an adhesive patch.

3. A receptacle according to Claim 1 or 2 wherein the receptacle has loop handles proximate to the inlet opening.

4. A receptacle according to any preceding claim wherein the securing means is shaped to describe a semi-circle around the bodily attachment.

5. A receptacle according to any preceding claim wherein the securing means is shaped to describe a "U" around the bodily attachment.

6. A receptacle according to any preceding claim wherein the securing means is shaped to describe a horizontal seal below a bodily attachment of the bodily waste collection bag shape.

7. A receptacle according to any preceding claim wherein the securing means forms vertical seals to the right-hand and left-hand side relative to the bodily attachment.

8. A receptacle according to any preceding Claim wherein the securing means takes the form of a belt which ties around the body of the end-user in addition to or in place of any adhesive strip.

9. A receptacle according to any preceding Claim wherein the receptacle is in the form of a container for holding the bodily waste materials, a bodily waste material collection bag, its bodily attachment and associated wipes received upon changing of the bodily waste material collection bag and its bodily attachments.

10. A receptacle according to any preceding Claim wherein the receptacle is collapsible from an expanded state for use to receive the discharged waste materials to a collapsed state for storage.

11. A receptacle according to Claim 10 wherein the collapsed state of the receptacle is a substantially flat condition thereof.

12. A receptacle according to any preceding Claim wherein the elongate body of the receptacle becomes narrower in a direction from the top end thereof towards the base end thereof.

13. A receptacle according to any preceding Claim wherein the elongate body of the receptacle does not become narrower in a direction from the top end thereof towards the base end thereof.

14. A receptacle according to any preceding Claim further comprising a closure for its inlet opening.

15. A receptacle according to any preceding claim wherein the receptacle is provided with one or more constrictions between the top end and the base end, the constriction preventing full expansion of the receptacle at a position intermediate the top end and the base end.

16. A receptacle according to Claim 15 wherein said constriction is formed by a weld.

17. A receptacle according to Claim 15 or Claim 16 wherein said constriction is formed by joining a front wall of the receptacle to a rear wall thereof.

18. A method for disposing of bodily waste materials comprising the steps of:
(i) attaching a receptacle to the skin in close proximity to a bodily waste material collection bag, its bodily attachment and an artificial excretory opening, so that an inlet opening of the receptacle receives the bodily waste discharge from an active artificial excretory opening;
(ii) placing the bodily waste material collection bag and its bodily attachments into the receptacle while the receiving receptacle is attached to the users body;
(iii) placing any wipes associated with the changing of the bodily waste material collection bag and its bodily attachment into the receptacle while the receiving receptacle is attached to the users body.

19. A kit comprising:
a receptacle with an elongate body with a top end, a base end, an intermediate portion between the top end and the base end, and formed with an internal bodily waste material-receiving chamber; an inlet opening formed on the body, proximate to, or at, the top end thereof, the body being adapted to receive within the inlet opening the bodily waste discharge from an active artificial excretory outlet; a bodily waste material collection bag, its bodily attachment and the contents of the bodily waste collection bag; and associated wipes, as used in the changing of bodily waste material collection bags, for placing into the receptacle while the receptacle is attached to the skin in close proximity to an artificial excretory opening.
